# EUROPEAN PATENT APPLICATION

(11) **EP 1 205 548 A1**
(43) Date of publication of application: **15.05.2002**
(21) Application number: 01126319.1
(22) Date of filing: 06.11.2001
(51) Int. Cl.: C12N 15/10, C07H 21/00

(54) **Methods and apparatus for successively ligating double-stranded DNA molecules on a solid phase**

(30) Priority: 09.11.2000 JP 2000342002
(71) Applicant: Kabushiki Kaisha Toyota Chuo Kenkyusho, Aichi-gun, Aichi-ken, 480-1192 (JP); Nara Institute of Science and Technology, Ikoma, Nara 630-0101 (JP)
(72) Inventor: Shinmyo, Atsuhiko,, Ikoma-gun, Nara 636-0151 (JP); Shibata, Daisuke, Kisarazu-shi, Chiba 292-0814 (JP); Kohda, Katsunori, K. K. Toyota Chuo Kenkyusho, Aichi-gun, Aichi 480-1192 (JP)
(74) Representative: Schmidtchen, Jürgen Christian

(57) **Abstract**

An object of this invention is to provide a method that enables successive ligation of many DNA molecules while specifying the ligation orientation without cloning, to produce a DNA molecule in which many DNA molecules are ligated by using this method, and to provide a use for the DNA molecule obtained by using this method.

Amethod to ligate many DNA molecules was developed by utilizing a solid phase for ligating DNA molecules that enables successive ligation of many DNA molecules without cloning like that in the liquid phase. In addition, the method allowed specification of the ligation orientation of the DNA molecules, by using non-palindromic sequences at the protruding ends of the DNA molecules to be ligated.

## Description

### FIELD OF THE INVENTION

This invention relates to a method for successively ligating double-stranded DNA molecules on a solid phase, a method for producing double-stranded DNA molecules successively ligated, and use of the double-stranded DNA molecules obtained using these methods.

### BACKGROUND OF THE INVENTION

When trying to enhance traits of organisms such as plants, animals, and microorganisms, and to impart new traits to these organisms, introduction of a DNA fragment encoding only a single gene is often insufficient to accomplish the initial object. In these cases, ligation of multiple genes prior to introduction of the genes into the organism is effective. In addition, introduction of multiple DNA fragments ligated to each other is effective, when a DNA fragment encoding an antisense RNA corresponding to a gene involved in metabolic system is introduced into an organism in order to repress the metabolic system of the organism. Accordingly, as one of many biotechnological techniques, development of techniques to ligate multiple DNA fragments is being attempted.

Conventionally, when ligating multiple DNA fragments, cycles of following steps (1) to (3) were usually repeated for each DNA fragment:
(1) performing recombination reaction of DNA fragments in liquid phase;
(2) forming palindromic ends or blunt ends on the DNA fragments by digestion of DNA fragment ends with restriction enzymes and ligating between the DNA fragments; and
(3) cloning the ligated DNA fragments into *E. coli* and then determining by restriction enzyme treatment whether the DNA fragment of interest is inserted into the cloned strain.

However, such operation not only was complicated but also required a lot of time and effort, and, moreover, it was difficult to stably maintain the ligated DNA fragments during this cycle. Furthermore, when palindromic ends or blunt ends were used as the ligation sites of the DNA fragments, the problem that the ligation orientation of the DNA fragment was not specified to one orientation occurred, resulting in a recombinant ligated in the reverse orientation.

Therefore, development of a method to define the ligation orientation of DNA fragments was attempted. For example, Unexamined Published Japanese Patent Application No. (JP-A) Hei 9-9979 discloses a method for defining the ligation orientation of DNA fragments. In this method, a fragment carrying a recognition site for SfiI, an enzyme that reacts with a DNA fragment to produce a non-palindromic sequence on its end, and a recognition site for other enzymes is inserted into a plasmid that has a SfiI recognition site, and then a plasmid that has one SfiI recognition site is produced. In addition, US Patent No. 5,595,895 discloses a method for producing a vector that enables cloning in one orientation by insertion of two SfiI cleavage sites into the vector using adapters, and such. Cleavage of this vector at SfiI site causes formation of protruding ends with non-palindromic sequences at both ends. For example, in the case of cloning a cDNA into this vector in a particular orientation, an adapter having a SfiI cleavage site is added to both ends of the cDNA, and then, the cDNA is treated with Sfi and ligated to a cloning vector which has also been treated with SfiI.

However, when ligating multiple DNA fragments using these liquid phase methods , in order to define the orientation of ligation, two or more restriction enzyme sites are required on one of the ligation fragments. In addition, because a cloning operation is required for every DNA fragment ligation operation, the complexity of the procedure is not at all improved. Thus, in order to ligate many DNA fragments using this method, a lot of time and effort will be required. Furthermore, these gazettes only disclose ligation between two DNA fragments, and thus, the methods described in these gazettes do not allow successive ligation of many DNA fragments without cloning.

On the other hand, with regard to ligation of DNA fragment using a solid phase system, a method is described in JP-A Hei 5-260972. However, only the ligation of two DNA fragments are disclosed in the publication, and in the method described, since the ligation of DNA fragments uses a palindromic sequence, the problem of not being able to define the ligation orientation still remained.

### SUMMARY OF THE INVENTION

In view of such situation, this invention was performed. An objective of this invention is to provide a method that allows multiple DNA molecules to be successively ligated without cloning with the ligation orientation defined. Also, an objective of this invention is to produce a DNA molecule formed by ligating multiple DNA molecules using the method. Furthermore, an objective of this invention is to provide use of the DNA molecule obtained by using this method.

The present inventors made every effort to solve the above-mentioned issues and, as a result, discovered that ligating DNA molecules using non-palindromic sequences in a solid phase system enables successively ligating DNA molecules without cloning of DNA molecules with ligation orientation defined.

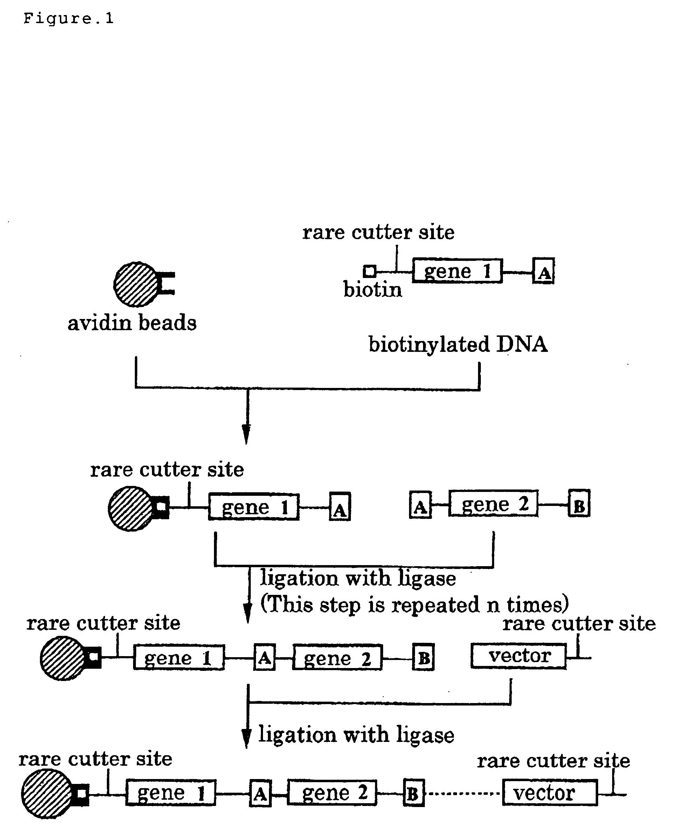

The mechanism of this invention is described as follows (it is indicated in FIG. 1). That is, first, DNA molecules whose protruding ends comprise non-palindromic sequences are immobilized onto a solid phase. Next, DNA molecules both protruding ends of which comprise non-palindromic sequences are added to a reaction system to allow contact the DNA molecules immobilized to the solid phase, and then by ligase reaction, these DNA molecules are ligated to each other. Then, DNA molecules unreacted with the DNA molecule immobilized to the solid phase are removed by washing. Depending on the number of DNA molecules to be ligated, a cycle consisting of addition of DNA molecules to the reaction system, ligase reaction, and washing is repeated. Furthermore, when the ligated DNA molecules is to be obtained, the DNA molecules are removed from the solid support by restriction enzyme treatment and so on.

Conventional ligation methods of DNA molecules in a liquid phase system did not allow removal of unreacted DNA from the reaction system, thus decreasing the ligation efficiency of DNA molecules, and association between DNA molecules that have complementary ligation sites was difficult when ligating many DNA molecules. Therefore, in the case of ligation of multiple DNA molecules, it was necessary to clone the DNA molecules. By using a solid phase system for ligating DNA molecules, the method of this invention enables successive ligation of multiple DNA molecules without cloning, as required in ligation in the liquid phase system. Furthermore, by using non-palindromic sequences for the protruding ends of the ligating DNA molecules, this method enables defining the ligation orientation of DNA molecules. Therefore, the method of the present invention allows enhancing the successive ligation efficiency of multiple DNA molecules remarkably.

That is, this invention relates to a method for successive ligation of DNA molecules in a solid phase system using non-palindromic sequences, and more specifically provides the following:
(1) A method for successively ligating double-stranded DNA molecules, the method comprising the steps of:
   (a) binding, to a solid phase, a double-stranded DNA molecule having a protruding end that is a non-palindromic sequence;
   (b) ligating, to the double-stranded DNA molecule bound to the solid phase, a double-stranded DNA molecule having, at both ends of it, protruding ends that are non-palindromic sequences;
   (c) eliminating, from a reaction system comprising the solid phase, the double-stranded DNA molecule having the protruding ends at both ends of it, wherein the double-stranded DNA molecule having the protruding ends is unreacted with the double-stranded DNA molecule bound to the solid phase; and
   (d) repeating the steps (b) and (c) as necessary.
(2) The method of (1), wherein, in the step (a) , one of two molecules having affinity for each other is bound to the double-stranded DNA molecule, the other is bound to the solid phase, and the double-stranded DNA molecule is bound to the solid phase using the affinity.
(3) The method of (1) or (2), wherein the two molecules having affinity for each other are avidin and biotin.
(4) The method of any one of (1) to (3), wherein, in the step (b) , the double-stranded DNA molecules are ligated to each other by ligase reaction.
(5) The method of any one of (1) to (4) , wherein the non-palindromic sequence in the protruding end of the double-stranded DNA molecule is formed by restriction enzyme treatment.
(6) The method of (5), wherein the restriction enzyme is selected from the group consisting of SfiI, BstXI, BbsI, BbvI, BglI, BsaI, BSmAI, BsmBI, BsmFI, BspMI, BstAPI, DraIII, Earl, FokI, HgaI, PfiMI, SfaNI, and Van91I.
(7) The method of any one of (1) to (6), wherein three or more double-stranded DNA molecules are successively ligated.
(8) A method for producing successively ligated double-stranded DNA molecules, the method comprising the steps of:
   (a) binding, to a solid phase, a double-stranded DNA molecule having a protruding end that is a non-palindromic sequence;
   (b) ligating, to the double-stranded DNA molecule bound to the solid phase, a double-stranded DNA molecule having, at both end of it, protruding ends that are non-palindromic sequences;
   (c) eliminating, from the reaction system comprising the solid phase, the double-stranded DNA molecule having the protruding ends at both ends of it, wherein the double-stranded DNA molecule having the protruding ends is unreacted with the double-stranded DNA molecule bound to the solid phase;
   (d) repeating the steps (b) and (c) as necessary; and
   (e) separating, from the solid phase, the double-stranded DNA molecules bound to the solid phase.
(9). The method of (8), wherein, in the step (a) , one of two molecules having affinity for each other is bound to the double-stranded DNA molecule, the other is bound to the solid phase, and the double-stranded DNA molecule is bound to the solid phase using the affinity.
(10) The method of (8) or (9), wherein the two molecules having affinity for each other are avidin and biotin.
(11) The method of any one of (8) to (10), wherein, in the step (b), the double-stranded DNA molecules are ligated to each other by ligase reaction.
(12) The method of any one of (8) to (11), wherein the non-palindromic sequence in the protruding end of the double-stranded DNA molecule is formed by restriction enzyme treatment.
(13) The method of (12) , wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.
(14) The method of any one of (8) to (13), wherein three or more double-stranded DNA molecules are successively ligated.
(15) A double-stranded DNA molecule produced by the method of any one of (8) to (14).
(16) A vector containing the double-stranded DNA molecule of (15).
(17) A transformant carrying the double-stranded DNA molecule of (15) or the vector of (16).
(18) A polypeptide encoded by the double-stranded DNA molecule of (15).
(19) A method for producing the polypeptide of (18), the method comprising the steps of cultivating the transformant of (17), and collectingan expressed polypeptide from the transformant or a culture supernatant thereof.
(20) An apparatus for successively ligating double-stranded DNA molecules, the apparatus comprising:
   (a) a solid phase to which a double-stranded DNA molecule having a protruding end that is a non-palindromic sequence is bound;
   (b) a reaction vessel retaining the solid phase;
   (c) multiple vessels each independently reserving (i) a liquid phase comprising a double-stranded DNA molecule having a protruding end that is a non-palindromic sequence, (ii) at least one or more liquid phases comprising a double-stranded DNA molecule having, at both ends of it, protruding ends that are non-palindromic sequences, (iii) a solution comprising an enzyme, and (iv) a washing liquid;
   (d) a means for transferring the solid phase, the means recovering the solid phase from one of the vessels (b) and (c) and transferring the solid phase to another of the vessels (b) and (c); and
   (e) a means for regulating drive of the means for transferring the solid phase.
(21) The apparatus of (20), wherein the solid phase is a magnetic bead and the means for transferring the solid phase is a magnet.
(22) The apparatus of (20) or (21), wherein the non-palindromic sequence of the protruding end of the double-stranded DNA molecule is formed by restriction enzyme treatment.
(23) The apparatus of (22), wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.
(24) An apparatus for successively ligating double-stranded DNA molecules, the apparatus comprising:
   (a) a solid phase to which a double-stranded DNA molecule having a protruding end that is a non-palindromic sequence is bound;
   (b) a reaction vessel retaining the solid phase and comprising an inlet and an outlet for a liquid phase;
   (c) a liquid phase reservoir each independently reserving (i) a liquid phase comprising a double-stranded DNA molecule having a protruding end that is a non-palindromic sequence, (ii) at least one or more liquid phases comprising a double-stranded DNA molecule having, at both ends of it, protruding ends that are non-palindromic sequences, (iii) a solution comprising an enzyme, and (iv) a washing liquid, the liquid phase reservoir comprising an open and close valve that regulates outflow of each of the liquid phases;
   (d) aliquid-phase-supplying path connecting the inlet of the reaction vessel and the open and close valve of the liquid phase reservoir, and a liquid-phase-draining path connected to the outlet of the reaction vessel;
   (e) an open and close valve that is set up at a position along the draining path and that regulates opening and closing of the draining path;
   (f) a means for driving the liquid phase, the means being set up at a position along the draining path; and
   (g) a means for regulating the open and close valve of the liquid phase reservoir and the open and close valve set up along the draining path, and the means for driving the liquid phase.
(25) An apparatus for successively ligating double-stranded DNA molecules, the apparatus comprising:
   (a) a solid phase to which a double-stranded DNA molecule having a protruding end that is a non-palindromic sequence is bound;
   (b) a reaction vessel retaining the solid phase and comprising an inlet and an outlet for a liquid phase;
   (c) a liquid phase reservoir each independently reserving (i) a liquid phase comprising a double-stranded DNA molecule having a protruding end that is a non-palindromic sequence, (ii) at least one or more liquid phases comprising a double-stranded DNA molecule having, at both ends of it, protruding ends that are non-palindromic sequences, (iii) a solution comprising an enzyme, and (iv) a washing liquid, the liquid phase reservoir comprising an open and close valve that regulates outflow of each of the liquid phases;
   (d) a liquid-phase-supplying path connecting the inlet of the reaction vessel and the open and close valve of the liquid phase reservoir, a liquid-phase-draining path connected to the outlet of the reaction vessel, and a circulating path connecting the inlet and the outlet of the reaction vessel;
   (e) a path-switching valve that is set up at a position along the draining path and that enables alternative connection between the circulating path and the draining path;
   (f) a means for driving the liquid phase, the means being set up at a position along the draining path between the outlet of the reaction vessel and the path-switching valve; and
   (g) a means for regulating the means for driving the liquid phase, together with the open and close valve and the path-switching valve.
(26) The apparatus of (24) or (25), wherein the solid phase is a bead and the reaction vessel has a column structure.
(27) The apparatus of any one of (24) to (26), wherein the means for driving the liquid phase is a pump.
(28) The apparatus of any one of (24) to (27), wherein the means for regulating regulates the open and close valve, the path-switching valve, and an operation time of the means for driving the liquid phase.
(29) The apparatus of any one of (24) to (28), wherein the double-stranded DNA molecule bound to the solid phase has a cleavage site for cleaving a bond to the solid phase and wherein the apparatus further comprises a means for cleaving a ligated double-stranded DNA molecule from the solid phase by acting on the cleavage site.
(30) The apparatus of (29) , wherein the double stranded DNA molecule bound to the solid phase comprises an S-S bond at the cleavage site and wherein the means for cleaving is a means for circulating, in the reaction vessel, a reducing agent cleaving the S-S bond.
(31) The apparatus of (29), wherein the double stranded DNA molecule bound to the solid phase comprises, at the cleavage site, a molecule that is cleaved by irradiation of light at a designated wavelength and wherein the means for cleaving is a means for irradiating light at the designated wavelength to the solid phase.
(32) The apparatus of any one of (24) to (31), further comprising, downstream of the draining path, a means for electrophoresis, wherein drained liquid is supplied to the means for electrophoresis.
(33) The apparatus of any one of (24) to (32), wherein the non-palindromic sequence of the protruding end of the double-stranded DNA molecule is formed by restriction enzyme treatment.
(34) The apparatus of (33) , wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.
(35) A reaction vessel for successively ligating double-stranded DNA molecules, the reaction vessel retaining an avidinylated bead, wherein a double-stranded DNA molecule having a protruding end that is a non-palindromic sequence is bound to the bead via a biotin molecule and a cleavage site.
(36) The reaction vessel of (35), wherein the double-stranded DNA molecule bound to the bead comprises an S-S bond at the cleavage site.
(37) A reaction vessel for successively ligating double-stranded DNA molecules, the reaction vessel retaining a silane-coated bead, wherein a double-stranded DNA molecule having a protruding end that is a non-palindromic sequence is amide-bonded to the bead via a cleavage site.
(38) The reaction vessel of (37), wherein the double-stranded DNA molecule amide-bonded to the bead comprises, at the cleavage site, a molecule that is cleaved by irradiation of light at a designated wavelength.
(39) The reaction vessel of any one of (35) to (38), wherein the reaction vessel has a column structure having an inlet and an outlet for a liquid phase.
(40) The reaction vessel of any one of (35) to (39), wherein the non-palindromic sequence of the protruding end of the double-stranded DNA molecule is formed by restriction enzyme treatment.
(41) The reaction vessel of (40), wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.

In the present invention, "palindromic sequence" refers to a nucleotide sequence whose complementary strand is an identical nucleotide sequence with itself. For example, "5'ACGT" is palindromic sequence because the complementary strand of it is "5'ACGT". On the other hand, in this invention, "non-palindromic sequence" refers to a nucleotide sequence, whose complementary strand is a different nucleotide sequence. For example, "5'ATCG" is non-palindromic sequence because the complementary strand of it is "5'CGAT" and they are not identical to each other. In addition, when a nucleotide sequence comprising an odd number of bases, the nucleotide sequence will always be a non-palindromic sequence, regardless of the type of bases (A. C. G. T.).

### BRIEF DESCRIPTION OF THE DRAWINGS

FIG. 1 shows the mechanism of the multiple gene ligation technology of the present invention. In the figure, A and B indicate palindromic sequences for SfiI, etc.
FIG. 2 shows the steps of ligating two gene fragments on a solid phase utilizing non-palindromic sequences.
FIG. 3 is an electrophoregram indicating the results of ligation of two or three gene fragments on a solid phase utilizing non-palindromic sequences. Lane 1 shows the marker, lane 2 shows the results of ligating two gene fragments, and lane 3 shows the results of ligating 3 gene fragments.
FIG. 4 shows the steps of ligating three gene fragments on a solid phase utilizing non-palindromic sequences.
FIG. 5 shows the steps of ligating four gene fragments on a solid phase utilizing non-palindromic sequences.
FIG. 6 is an electrophoregram indicating the results of ligating four gene fragments on a solid phase utilizing non-palindromic sequences. Lane 1 shows the marker, and lane 2 shows the results of ligating four gene fragments.
FIG. 7 is an electrophoregram indicating the results of the ligation of four gene fragments in a column on a solid phase using non-palindromic sequences. Lane 1 shows the marker and lane 2 shows the results of ligation of four gene fragments.
FIG. 8 is an electrophoregram indicating the results of immobilization to a solid phase in a circular column using non-palindromic sequences.
FIG. 9 is an electrophoregram indicating the results of ligation of gene fragments to a solid phase in a circular column using non-palindromic sequences.

### DETAILED DESCRIPTION OF THE INVENTION

This invention provides a novel method for successively ligating double-stranded DNA molecules. First, the double-stranded DNA molecule, whose protruding end has a non-palindromic sequence, is bound to a solid phase (step (a)).

For example, restriction enzyme treatment can be used as a method for providing non-palindromic sequences at the ends of a double-stranded DNA molecule. There is no particular limitation on the restriction enzyme, as long as it produces non-palindromic sequences at the protruding ends of the digested double-stranded DNA molecule. Examples of such restriction enzymes include, but are not limited to, for example, SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I. As an example of another method for producing the non-palindromic sequences at the ends of a double-stranded DNA molecule, addition of an adapter having non-palindromic sequences at its ends to the ends of a double-stranded DNA molecule is used.

As an example of a further method for producing the non-palindromic sequences at the ends of a double-stranded DNA molecule, T4DNA polymerase treatment is used. In this method, a non-palindromic sequence in the protruding end is formed through removal of bases up to the base preceding the first A or G base from the end by the action of exonuclease of T4 polymerase (See LIC vector kits, TAKARA).

A method utilizing two molecules having affinity for each other can be used as an example of a method for ligating a double-stranded DNA molecule to a solid phase. One of these molecules is bound to a solid phase, the other is bound to a double-stranded DNA molecule, and therefore, the double-stranded DNA molecule and the solid phase can be bound by the affinity between these molecules. As molecules that have such mutual affinity, for example, an avidin-biotin system may be used preferably, but examples are not limited thereto. In the ligation of the double-stranded DNA molecules to a solid phase, it is also possible to use a method in which the ends of the double-stranded DNA are aminated and the DNA is amide-bonded to the solid phase (for example, polystyrene plate, silane-coated glass, and such).

Next, in this invention, the double stranded DNA molecules, in which both protruding ends are non-palindromic sequences, are ligated to the double-stranded DNA molecules bound to the solid phase (step (b)).

Ligation between double-stranded DNA molecules can be carried out by utilizing a ligase reaction. There are no particular limitations on the enzymes that can be used for ligase reactions as long as they can ligate DNA molecules to each other, and, for example, T4DNA ligase can be used preferably. It is also possible to use Taq and Pfu, which are thermostable enzymes, and *E. coli* ligase. Ligase reaction that utilizes T4DNA ligase is usually carried out in the presence of Mg, DTT, and ATP, using Tris-HCl (pH 7.6) as a buffer. Furthermore, reaction efficiency can be enhanced by addition of PEG. The reaction is usually carried out at 16°C for 2 hours or longer. Commercially available products (for example, DNA Ligation kit from TAKARA) may be also used as reagents for the ligase reaction. Besides ligases, recombinase (Lifetech) and TOPO isomerase I (Invitrogen) may be used to ligate double-stranded DNA molecules to each other.

Next, in the method of this invention, double-stranded DNA molecules that have unreacted with the double-stranded DNA molecules bound to the solid phase are eliminated from the reaction system (step (c)).

In this step, first, the double-stranded DNA molecules bound to the solid phase can be separated from the reaction solution containing unreacted double-stranded DNA molecules. Next, the buffer can be added to the double-stranded DNA molecule bound to the solid phase. For example, when magnetic beads are used as the solid phase, after ligase reaction, these beads can be collected using the magnetic force of a magnet followed by the removal of the supernatant containing the unreacted double-stranded DNA. Next, buffer (for example, T.E. buffer, and such) can be added to the beads, then the magnet can be moved away from the magnetic beads to free the magnetic beads from the magnetic force, and the magnetic beads can then be gently suspended into the buffer. Removal of the supernatant and addition of buffer are repeated as necessary.

In this invention, depending on the number of double-stranded DNA molecules to be ligated, the above-mentioned steps (b) and (c) can be repeated as necessary (step (d)). This enables successive ligation of many double-stranded DNA molecules.

Moreover, the double-stranded DNA molecules bound to the solid phase are separated from the solid phase when successively ligated double-stranded DNA molecules using the DNA fragment ligation method of this invention are to be obtained (step (e)).

Separation of DNA molecules from the solid phase can be carried out by restriction enzyme treatment. For example, when using magnetic beads as the solid phase, first, restriction enzyme treatment is carried out with the double-stranded DNA immobilized onto the magnetic beads. This treatment separates the double-stranded DNA from the magnetic beads and releases them into solution. After restriction enzyme treatment, the magnetic beads are collected using the magnetic force of a magnet, and the supernatant is recovered. In this way, the double-stranded DNA molecule of interest in the supernatant can be obtained.

When avidin-biotin system is used for binding double-stranded DNA molecules to a solid phase, properties of the biotin molecules attached to the double-stranded DNA molecules can be utilized for separating the double-stranded DNA molecules from the solid phase. In the double-stranded DNA molecule bound to the solid phase, usually, an S-S bond that dissociated by treatment with reducing agents such as DTT, or molecules that dissociated by light irradiation at a certain wavelength are used to connect the spacer and the biotin molecule. In this way, after ligation, the double-stranded DNA of interest can be separated from the solid phase by treatment of reducing agent or by light irradiation.

It is possible to carry out all the steps as above in the method of the present invention by hand, but it is also possible to automate all or parts of them using a device for carrying out the steps. The present invention includes a device for carrying out the method of the above-mentioned invention.

The double-stranded DNA molecule obtained by the method of this invention can be used to produce fusion polypeptides by fusing polypeptides encoded by multiple double-stranded DNA molecules from which the double-stranded DNA molecule of this invention are derived.

In the production of fusion polypeptides, for example, the double-stranded DNA molecule obtained by the method of this invention can be further fused with a DNA molecule encoding a GST or His-tag, and then, its recombinant polypeptide expressed can be easily purified using an affinity column that contains, as carriers, ligands that bind to GST and His-tag. When fusing the double-stranded DNA molecule obtained by the method of this invention with the DNA molecule encoding GST and His-tag, PET vector into which a His-tag (Novagen) is inserted and a pGEX vector for fusion with GST (Pharmacia) may be used. *E. coli* may be used as a host to be introduced the above-mentioned vector into which double-stranded DNA molecule is inserted.

In addition, by using green fluorescent protein (GFP) and luciferase (Luc) as genes to be fused with double-stranded DNA molecules, it is possible to construct a fusion polypeptide reporter system for microorganisms, plants, and animals. As vectors to be used for this reporter system, for example, pQBI25 (TAKARA) and pQBI63 (TAKARA) when GFP is used as a reporter, pGL vector (Promega) when luciferase is used as a reporter, and such may be used. For example, animal cells and *E. coli* may be used preferably as the host to be introduced the vectors. When plant cells are used as the host, pBI101.2 may be used as vectors preferably.

In addition, the double-stranded DNA molecule obtained by the method of this invention can be applied to the production of transgenic organisms. When trying to enhance the traits of each organism such as plants, animals, and microorganisms, or to add new traits to these organisms, the introduction of a DNA fragment encoding only one gene is insufficient to achieve the initial object. In this case, ligation of multiple genes prior to introduction of the genes into the organism is effective. Furthermore, even when introducing a DNA fragment that encodes antisense RNA corresponding to the gene involved in the metabolic system to repress the metabolic system of the organism, ligation of multiple DNA fragments prior to introduction of the fragments into the organism is effective. According to the method of this invention, a series of gene groups can be multiply ligated efficiently, and by introduction of them into each of the organisms such as plants, animals and microorganisms, the traits of the organisms can be altered effectively.

In production of transgenic plants, for example, first, a callus is induced, and then a vector containing the gene of interest is introduced into the callus through *Agrobacterium* method, particle gun method, etc. Next, the effect of drug-resistant gene inserted into the vector (for example, resistance to kanamycin or hygromycin) is used as an index to select the strain into which a vector has been inserted. Subsequently, hormones are added to them for differentiation the callus to obtain the regenerated plant. In this method, to obtain the regenerated plant in one turn, at least about 2 months is required, and if the above-mentioned method is repeated to introduce many genes, a great deal of time will be required. Therefore, by using genes multiply ligated through the method of the present invention, transgenic plants into which many genes are inserted can be produced in a short period of time.

When producing transgenic animals, usually, the gene of interest in the form of a linear DNA is introduced into the nucleus of a fertilized egg. Also in this case, by using genes multiply ligated by the method of this invention, transgenic animals into which many genes are inserted can be produced in a short period of time.

In addition, besides the purpose of adding new traits to an organism, the multiply ligated genes produced by the method of this invention can be used, for example, to produce a variety of substances within an organism.

Therefore, for use in the production of the above-mentioned fusion polypeptides or transgenic organisms, or in material production *in vivo*, the present invention includes a vector that contains a double-stranded DNA molecule obtained through the method of this invention, and a transformant that harbors the vector or the double-stranded DNA molecule. Furthermore, the present invention includes a method for producing recombinant polypeptides using the transformant, and the recombinant polypeptide that can be obtained from this method.

This invention enabled successive ligation of many DNA fragments, while defining the ligation orientation without cloning. Accordingly, ligation of many DNA fragments (for example, three or more DNA fragments) was facilitated, and the load on time and effort for it was remarkably reduced. For example, the method of the present invention is effective for application in ligation of many genes for transformation of an organism, in ligation of multiple DNA fragments for construction of a vector, and in construction of mutant genes.

Any patents, patent applications, and publications cited herein are incorporated by reference.

The present invention will be explained in detail below with reference to examples, but it is not to be construed as being limited thereto.

### EXAMPLE 1

### Immobilization of nucleic acid fragments

M-280 kilo-base binder kit available from Dynal was used to immobilize the nucleic acid fragments. Streptavidinylated beads (5 µL) were placed into an Eppendorf tube. After collected by magnetism, the beads were washed with 20µl of binding solution, and then resuspended in 20 µl of the same buffer. Afterwards, 20µl of biotinylated DNA was added and mixed well, and then it left to stand for 3 hours at room temperature.

### EXAMPLE 2

### Ligation on a solid phase

After immobilization of the nucleic acid fragments, washing was carried out twice with 40 µl of washing solution and twice with 200 µl of T.E. buffer. Next, 20 µl of ligated genes having non-palindromic sequences in its sticky ends was added, and then an equivalent amount of I enzyme solution of ligation kit ver. 2 (TAKARA) was added. The reaction was carried out at 16°C for 2 hours while suspended every 30 minutes. Later, washing was carried out twice with 200 µl of T.E. buffer, and twice with the buffer to be used for the next restriction enzyme treatment. The ligated genes were recovered from the beads by carrying out the restriction enzyme treatment on a 50-µl scale.

### EXAMPLE 3

### Ligation of nucleic acid fragment using non-palindromic sequences on a solid phase

The mechanism of ligation system of multiple nucleic acid fragment is shown in FIG. 1. First, a nucleic acid fragment, in which one of its protruding ends has a non-palindromic sequence, is immobilized. To this immobilized nucleic acid fragment, a nucleic acid fragment both ends of which have non-palindromic sequences is ligated with a ligase. The preparation of a nucleic acid fragment that has non-palindromic sequences may be achieved, for example, by the following method: PCR with a primer having a cleavage site for a restriction enzyme, such as SfiI, BstXI, and such. Otherwise, it can be produced by preparing a donor plasmid containing two restriction sites for SfiI, BstXI, and such, by cloning a fragment between these sites, and by cleaving it at the restriction sites for SfiI, BstXI, and such. The nucleic acid fragments unreacted with ligase is removed by washing so that they will not affect the next reaction. The operations described above are repeated by turns. Finally, the multiply ligated nucleic acid fragment is obtained by removing the immobilized nucleic acid fragments using restriction enzymes. This method enables ligation of multiple nucleic acid fragment by a so-called "cassette method", in which a group of genes to be inserted is ligated at a time and is then inserted into the vector. In addition, by ligating a vector to one end of the ligated fragment and by dissociating it from the beads using restriction enzymes followed by its self-cyclization, a plasmid that carries multiply ligated nucleic acid fragments can be produced at a time.

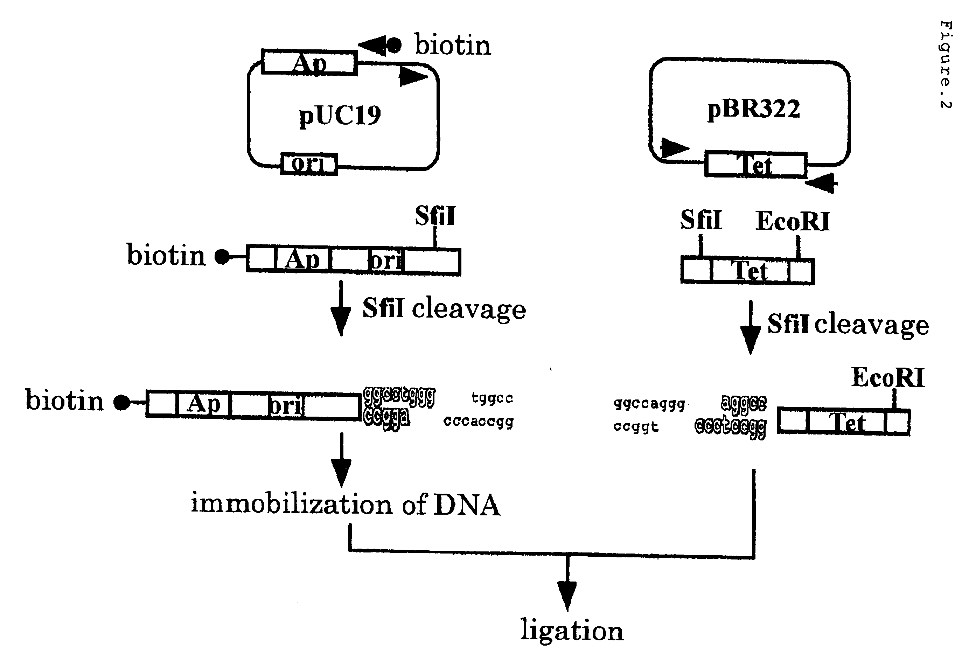

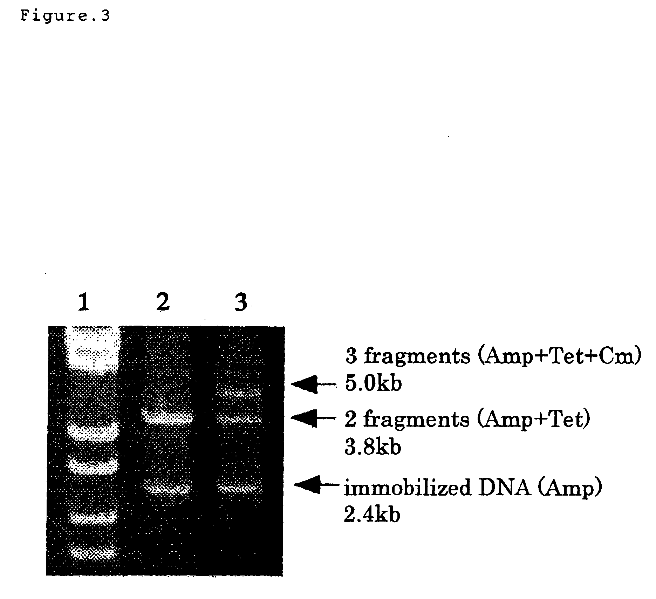

Regarding the ligation reaction on the solid phase through this series of operations, definition of the ligation orientation is thought to be important. Therefore, we attempted to define the ligation orientation by using a non-palindromic sequence for the ligation site. The ligation strategy is shown in FIG. 2. The sequence resulting from the use of restriction enzyme SfiI was selected as the ligation site. pUC19 was amplified as a form (approximately 2.6 kb) including the replication origin and an ampicillin resistant gene , using a biotinylated primer for one end and a primer containing SfiI restriction site for the other end. In addition, a tetracycline resistant gene of pBR322, selected as the gene to be ligated, was amplified (approximately 1.4 kb) by using a primer containing a SfiI cleavage site and a primer containing an EcoRI cleavage sequence as self-ligation sites. The primers were removed from the amplified fragments, using PCR purification kit (Stratagene), and cleavage was carried out with SfiI. When the ligated genes were collected from the beads by EcoRI treatment after immobilization and ligation of these fragments, a new band of approximately 3.8 kb was confirmed, in which two fragments were presumably ligated (FIG. 3). Following self-cyclization of this fragment, DH5α strain of *E. coli* was transformed with it and was spread onto L agar media supplemented with ampicillin and tetracycline. The obtained colony was treated with EcoRI restriction enzyme to confirm the chain length.

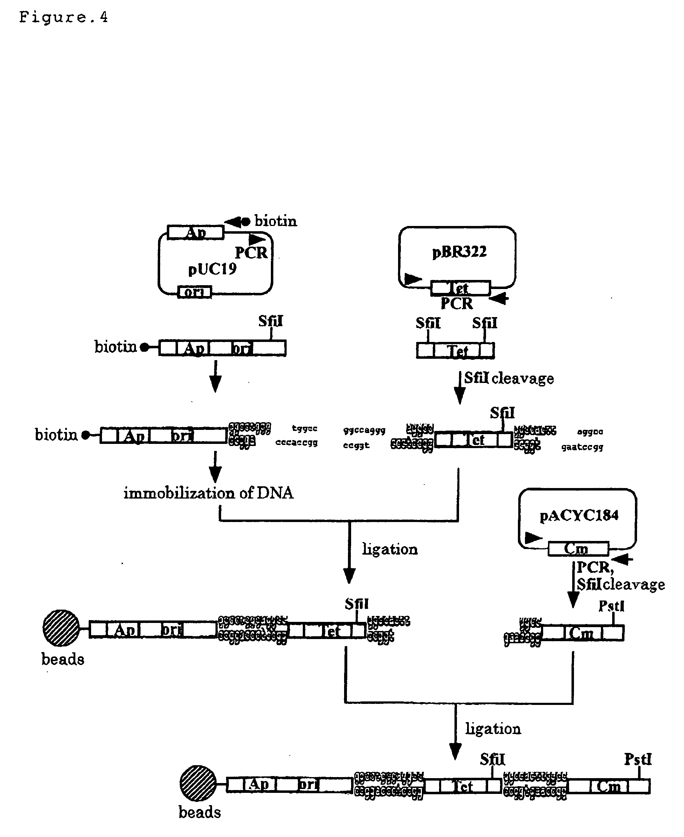

Next, a chloramphenicol resistant gene was ligated similarly. The strategy of multiple ligation is shown in FIG. 4. The tetracycline resistant gene was amplified by PCR using a primer having a SfiI restriction site. Protruding ends of different sequence were produced on the both ends of the amplified fragment by cleavage with SfiI. The chloramphenicol resistant gene on pACYC184 was amplified by PCR, and then cleaved with SfiI. Ligation on solid phase was performed using the fragment containing the replication origin and the ampicillin resistant gene, the fragment containing the tetracycline resistant gene, and the fragment containing the chloramphenicol resistant gene, described above. After ligation, when the ligated genes were cleaved with PstI restriction enzyme and recovered from beads, a fragment (5.0 kb) in which three fragments were ligated was confirmed (FIG. 3). After self-cyclization of this fragment, the DH5α strain of *E. coli* was transformed with it and spread onto L agar media supplemented with ampicillin and tetracycline. Furthermore, the obtained colony was spread onto L agar media supplemented chloramphenicol. Plasmids were obtained from the transformant strains and indicated that ligated genes were obtained by restriction enzyme treatment.

Furthermore, a kanamycin resistant gene on pACYC177 was similarly amplified by PCR so that its both ends carry SfiI sites, and treated with restriction enzyme (FIG. 4).

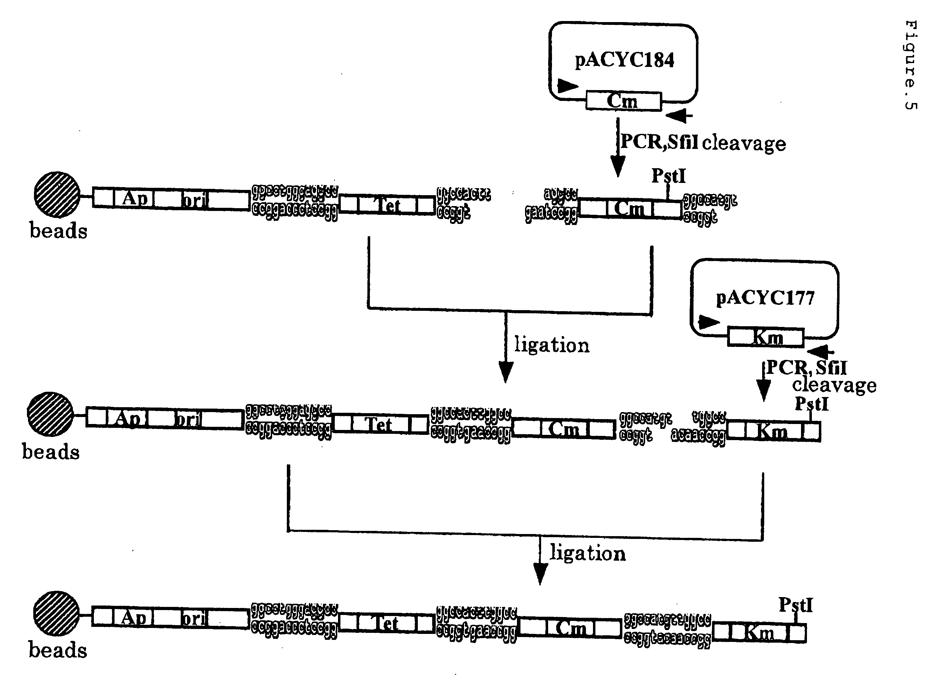

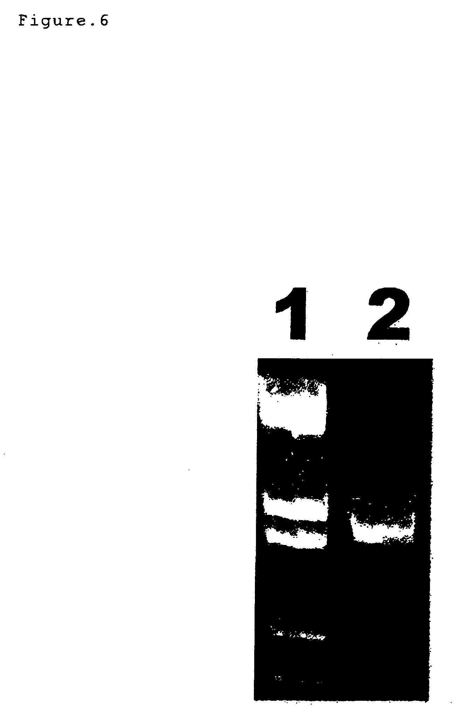

Furthermore, when gene ligation was carried out using four genes (7.5 kb), ampicillin, tetracycline, chloramphenicol and kanamycin resistant genes (FIG. 5), the ligated plasmid could be obtained. The obtained plasmids were treated with restriction enzyme PstI to confirm that ligated genes were obtained (FIG. 6).

Usually, when ligating two fragments, the ligation-cloning operation requires at least two days. In the case of four fragments, six days would be necessary. In contrast, this method, resulting a remarkable reduction in ligation time, requires only 6 hours for ligation. Furthermore, repetition of this step similarly enables ligation of more genes.

### EXAMPLE 4

To construct the above-mentioned apparatus, it is necessary to examine the successive ligation reactions of nucleic acid fragments in the column. As nucleic acid fragments to be ligated, those which were prepared by PCR and by subsequent BstXI treatment at the BstXI site added to the PCR primer were used. To prepare fragments to be immobilized, PCR was performed using a primer biotinylated at one end of it.

### EXAMPLE 5

Five µg of DNA to be immobilized was added to TBS+ 0.5 M NaCl buffer, the mixture was applied to a streptavidin-bound column (PIERCE), and the column was left to stand for 30 minutes. After standing, the column was washed and equilibrated by applying, to the column, 5 ml of TBS+ 0.5 M NaCl buffer three times and then 5 ml of ligase buffer. Then, 5 µg of the fragment to be ligated and 5 ml of ligase-containing solution were applied to the column and the ligation reaction was carried out at 16°C for 30 minutes. After the reaction, the solution was let to run off, then 5 µg of the fragment and 5 ml of ligase-containing solution were applied again to the column, and ligation reaction was carried out at 16°C for 30 minutes. This manipulation was repeated 4 times. After the ligation reaction, 5 ml of solution containing the second fragment and ligase was applied to the column and the ligation reaction was carried out similarly at 16°C for 2 hours. If necessary, the ligation reaction was repeated. After completion of the ligation reaction, 5 ml of TBS+ 0.5 M NaCl buffer was applied to the column 3 times for washing, and then 5 ml of buffer for restriction enzyme was applied to the column for equilibration. After equilibration, a restriction enzyme-containing solution was applied and reaction was carried out at 37°C for 2 hours. Then, 5 ml of TBS+ 0.5 M NaCl buffer was used for elution.

### EXAMPLE 6

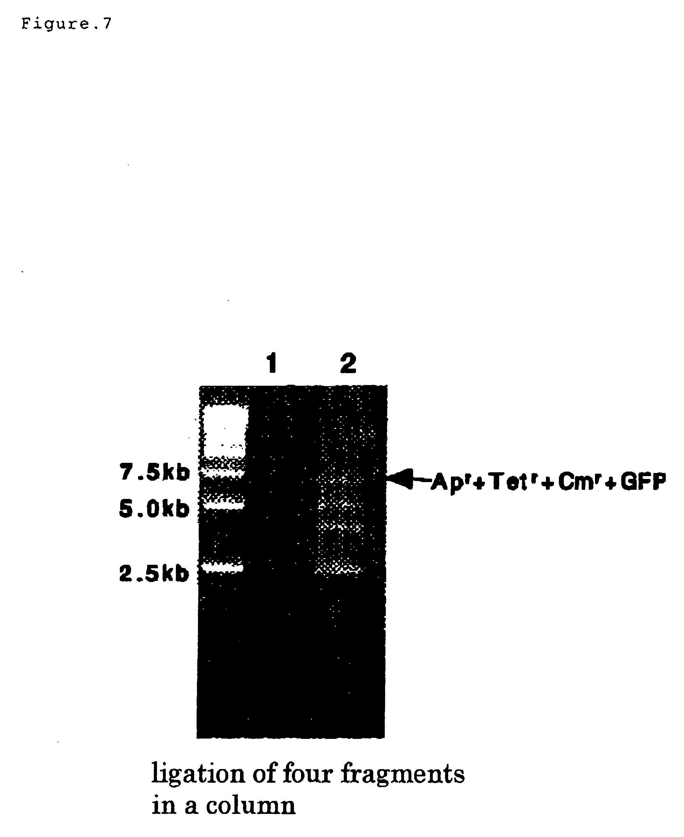

As the DNA to be immobilized, a region containing the replication origin of pUC19 and an ampicillin resistant gene was used. A cleaved protruding end containing a non-palindromic sequence was produced with BstXI restriction enzyme, and was immobilized on a column . Then, after cleavage by ISce-I restriction enzyme, the eluted fractions were subjected to electrophoresis to confirm that the DNA was immobilized within the column. When tetracycline resistant gene was selected as the gene to be ligated and ligation inside the column was performed, ligation of DNA fragments was confirmed. Then, chloramphenicol resistant gene and red shift Green fluorescent protein (rsGFP) gene were ligated. After ligation, cleavage with ISce-I and collection of it were carried out, and it was confirmed by electrophoresis that the genes were ligated. The band at the position of interest was collected, self-cyclized, and used for the transformation of *E. coli*. DNA was collected from a colony and ligation of DNA was confirmed by using restriction enzymes (FIG. 7).

### EXAMPLE 7

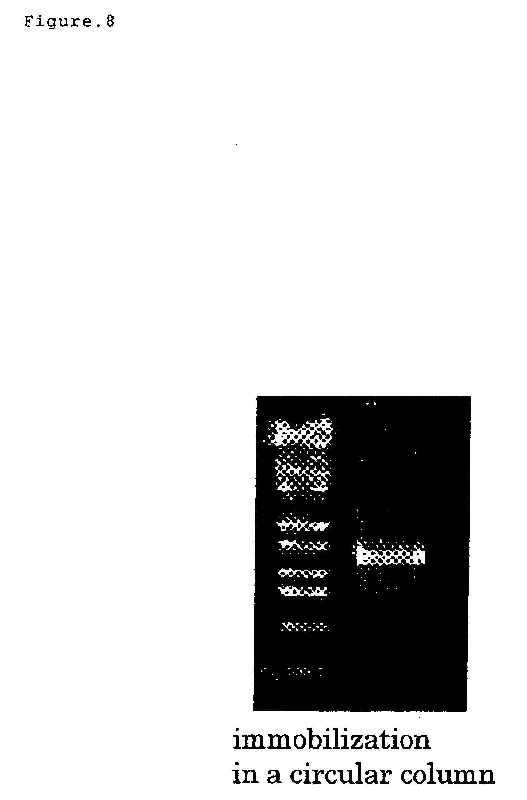

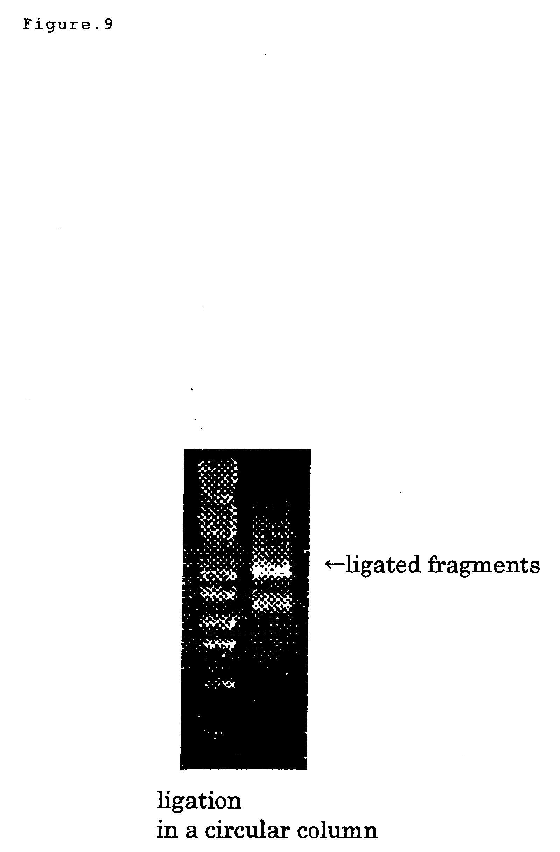

When constructing an apparatus, a system in which fractions eluted from a column are applied to the column again is desirable from the viewpoint of cost, utility, and efficiency. Therefore, a system to circulate the solution by a pump was constructed by placing a pathway made of silicone hose and such, from the outlet to the inlet of the column. The DNA to be immobilized and biotinylated pUC19 were applied to it, and by circulation for 30 minutes through a pump, immobilization was carried out. Next, the solution was eluted from the column, and the column was washed 3 times with 5 ml of TBS+ 0.5 M NaCl buffer and equilibrated using 5 ml of restriction enzyme buffer. Then, the solution containing restriction enzyme was applied to the column, followed by circulation by a pump. The reaction was performed at 37°C for 2 hours. After cleavage, elution was carried out with TBS+ 0.5 M NaCl buffer. The eluted solution was applied to electrophoresis, and its immobilization was confirmed (FIG. 8). In addition, after immobilization, the column was washed 3 times with 5 ml of TBS+ 0.5 M NaCl buffer and equilibrated with 5 ml of ligase buffer, and 5 ml of solution containing ligase and the fragment to be ligated to the column next were applied to the column. Then, the reaction was carried out at 16°C for 2 hours by circulation of the solution by a pump. After the reaction, the solution was eluted from the column, the column was equilibrated with 5 ml of ligase buffer, and 5 ml of solution containing 20 µg of tetracycline resistant gene cleaved with SfiI was added to the column. The reaction was carried out at 16°C for 2 hours with circulation by a pump. Consequently, ligated fragments were confirmed (FIG. 9).

## Claims

1. A method for successively ligating double-stranded DNA molecules, the method comprising the steps of:
(a) binding, to a solid phase, a double-stranded DNA molecule having a protruding end that is a non-palindromic sequence;
(b) ligating, to the double-stranded DNA molecule bound to the solid phase, a double-stranded DNA molecule having, at both ends of it, protruding ends that are non-palindromic sequences;
(c) eliminating, from a reaction system comprising the solid phase, the double-stranded DNA molecule having the protruding ends at both ends of it, wherein the double-stranded DNA molecule having the protruding ends is unreacted with the double-stranded DNA molecule bound to the solid phase; and
(d) repeating the steps (b) and (c) as necessary.

2. The method of claim 1, wherein, in the step (a) , one of two molecules having affinity for each other is bound to the double-stranded DNA molecule, the other is bound to the solid phase, and the double-stranded DNA molecule is bound to the solid phase using the affinity.

3. The method of claim 1 or 2, wherein the two molecules having affinity for each other are avidin and biotin.

4. The method of any one of claims 1 to 3, wherein, in the step (b), the double-stranded DNA molecules are ligated to each other by ligase reaction.

5. The method of any one of claims 1 to 4, wherein the non-palindromic sequence in the protruding end of the double-stranded DNA molecule is formed by restriction enzyme treatment.

6. The method of claim 5, wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.

7. The method of any one of claims 1 to 6, wherein three or more double-stranded DNA molecules are successively ligated.

8. A method for producing successively ligated double-stranded DNA molecules, the method comprising the steps of:
(a) binding, to a solid phase, a double-stranded DNA molecule having a protruding end that is a non-palindromic sequence;
(b) ligating, to the double-stranded DNA molecule bound to the solid phase, a double-stranded DNA molecule having, at both end of it, protruding ends that are non-palindromic sequences;
(c) eliminating, from the reaction system comprising the solid phase, the double-stranded DNA molecule having the protruding ends at both ends of it, wherein the double-stranded DNA molecule having the protruding ends is unreacted with the double-stranded DNA molecule bound to the solid phase;
(d) repeating the steps (b) and (c) as necessary; and
(e) separating, from the solid phase, the double-stranded DNA molecules bound to the solid phase.

9. The method of claim 8, wherein, in the step (a) , one of two molecules having affinity for each other is bound to the double-stranded DNA molecule, the other is bound to the solid phase, and the double-stranded DNA molecule is bound to the solid phase using the affinity.

10. The method of claim 8 or 9, wherein the two molecules having affinity for each other are avidin and biotin.

11. The method of any one of claims 8 to 10, wherein, in the step (b), the double-stranded DNA molecules are ligated to each other by ligase reaction.

12. The method of any one of claims 8 to 11, wherein the non-palindromic sequence in the protruding end of the double-stranded DNA molecule is formed by restriction enzyme treatment.

13. The method of claim 12 , wherein the restriction enzyme is selected from the group consisting of SfiI, BstXI, BbsI, BbvI, BglI, BsaI, BsrnAI, BsmBI, BsmFI, BspMI, BstAPI, DraIII, Earl, FokI, HgaI, PfiMI, SfaNI, and Van91I.

14. The method of any one of claims 8 to 13, wherein three or more double-stranded DNA molecules are successively ligated.

15. A double-stranded DNA molecule produced by the method of any one of claims 8 to 14.

16. A vector containing the double-stranded DNA molecule of claim 15.

17. A transformant carrying the double-stranded DNA molecule of claim 15 or the vector of claim 16.

18. A polypeptide encoded by the double-stranded DNA molecule of claim 15.

19. A method for producing the polypeptide of claim 18, the method comprising the steps of cultivating the transformant of claim 17, and collecting an expressed polypeptide from the transformant or a culture supernatant thereof.

20. An apparatus for successively ligating double-stranded DNA molecules, the apparatus comprising:
(a) a solid phase to which a double-stranded DNA molecule having a protruding end that is a non-palindromic sequence is bound;
(b) a reaction vessel retaining the solid phase;
(c) multiple vessels each independently reserving (i) a liquid phase comprising a double-stranded DNA molecule having a protruding end that is a non-palindromic sequence, (ii) at least one or more liquid phases comprising a double-stranded DNA molecule having, at both ends of it, protruding ends that are non-palindromic sequences, (iii) a solution comprising an enzyme, and (iv) a washing liquid;
(d) a means for transferring the solid phase, the means recovering the solid phase from one of the vessels (b) and (c) and transferring the solid phase to another of the vessels (b) and (c); and
(e) a means for regulating drive of the means for transferring the solid phase.

21. The apparatus of claim 20, wherein the solid phase is a magnetic bead and the means for transferring the solid phase is a magnet.

22. The apparatus of claim 20 or 21, wherein the non-palindromic sequence of the protruding end of the double-stranded DNA molecule is formed by restriction enzyme treatment.

23. The apparatus of claim 22, wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.

24. An apparatus for successively ligating double-stranded DNA molecules, the apparatus comprising:
(a) a solid phase to which a double-stranded DNA molecule having a protruding end that is a non-palindromic sequence is bound;
(b) a reaction vessel retaining the solid phase and comprising an inlet and an outlet for a liquid phase;
(c) a liquid phase reservoir each independently reserving (i) a liquid phase comprising a double-stranded DNA molecule having a protruding end that is a non-palindromic sequence, (ii) at least one or more liquid phases comprising a double-stranded DNA molecule having, at both ends of it, protruding ends that are non-palindromic sequences, (iii) a solution comprising an enzyme, and (iv) a washing liquid, the liquid phase reservoir comprising an open and close valve that regulates outflow of each of the liquid phases;
(d) a liquid-phase-supplying path connecting the inlet of the reaction vessel and the open and close valve of the liquid phase reservoir, and a liquid-phase-draining path connected to the outlet of the reaction vessel;
(e) an open and close valve that is set up at a position along the draining path and that regulates opening and closing of the draining path;
(f) a means for driving the liquid phase, the means being set up at a position along the draining path; and
(g) a means for regulating the open and close valve of the liquid phase reservoir and the open and close valve set up along the draining path, and the means for driving the liquid phase.

25. An apparatus for successively ligating double-stranded DNA molecules, the apparatus comprising:
(a) a solid phase to which a double-stranded DNA molecule having a protruding end that is a non-palindromic sequence is bound;
(b) a reaction vessel retaining the solid phase and comprising an inlet and an outlet for a liquid phase;
(c) a liquid phase reservoir each independently reserving (i) a liquid phase comprising a double-stranded DNA molecule having a protruding end that is a non-palindromic sequence, (ii) at least one or more liquid phases comprising a double-stranded DNA molecule having, at both ends of it, protruding ends that are non-palindromic sequences, (iii) a solution comprising an enzyme, and (iv) a washing liquid, the liquid phase reservoir comprising an open and close valve that regulates outflow of each of the liquid phases;
(d) a liquid-phase-supplyingpath connecting the inlet of the reaction vessel and the open and close valve of the liquid phase reservoir, a liquid-phase-draining path connected to the outlet of the reaction vessel, and a circulating path connecting the inlet and the outlet of the reaction vessel;
(e) a path-switching valve that is set up at a position along the draining path and that enables alternative connection between the circulating path and the draining path;
(f) a means for driving the liquid phase, the means being set up at a position along the draining path between the outlet of the reaction vessel and the path-switching valve; and
(g) a means for regulating the means for driving the liquid phase, together with the open and close valve and the path-switching valve.

26. The apparatus of claim 24 or 25, wherein the solid phase is a bead and the reaction vessel has a column structure.

27. The apparatus of any one of claims 24 to 26, wherein the means for driving the liquid phase is a pump.

28. The apparatus of any one of claims 24 to 27, wherein the means for regulating regulates the open and close valve, the path-switching valve, and an operation time of the means for driving the liquid phase.

29. The apparatus of any one of claims 24 to 28, wherein the double-stranded DNA molecule bound to the solid phase has a cleavage site for cleaving a bond to the solid phase and wherein the apparatus further comprises a means for cleaving a ligated double-stranded DNA molecule from the solid phase by acting on the cleavage site.

30. The apparatus of claim 29, wherein the double stranded DNA molecule bound to the solid phase comprises an S-S bond at the cleavage site and wherein the means for cleaving is a means for circulating, in the reaction vessel, a reducing agent cleaving the S-S bond.

31. The apparatus of claim 29, wherein the double stranded DNA molecule bound to the solid phase comprises, at the cleavage site, a molecule that is cleaved by irradiation of light at a designated wavelength and wherein the means for cleaving is a means for irradiating light at the designated wavelength to the solid phase.

32. The apparatus of any one of claims 24 to 31, further comprising, downstream of the draining path, a means for electrophoresis , wherein drained liquid is supplied to the means for electrophoresis.

33. The apparatus of any one of claims 24 to 32, wherein the non-palindromic sequence of the protruding end of the double-stranded DNA molecule is formed by restriction enzyme treatment.

34. The apparatus of claim 33, wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.

35. A reaction vessel for successively ligating double-stranded DNA molecules, the reaction vessel retaining an avidinylated bead, wherein a double-stranded DNA molecule having a protruding end that is a non-palindromic sequence is bound to the bead via a biotin molecule and a cleavage site.

36. The reaction vessel of claim 35, wherein the double-stranded DNA molecule bound to the bead comprises an S-S bond at the cleavage site.

37. A reaction vessel for successively ligating double-stranded DNA molecules, the reaction vessel retaining a silane-coated bead, wherein a double-stranded DNA molecule having a protruding end that is a non-palindromic sequence is amide-bonded to the bead via a cleavage site.

38. The reaction vessel of claim 37, wherein the double-stranded DNA molecule amide-bonded to the bead comprises, at the cleavage site, a molecule that is cleaved by irradiation of light at a designated wavelength.

39. The reaction vessel of any one of claims 35 to 38, wherein the reaction vessel has a column structure having an inlet and an outlet for a liquid phase.

40. The reaction vessel of any one of claims 35 to 39, wherein the non-palindromic sequence of the protruding end of the double-stranded DNA molecule is formed by restriction enzyme treatment.

41. The reaction vessel of claim 40, wherein the restriction enzyme is selected from the group consisting of SfiI, BstXI, BbsI, BbvI, BglI, BsaI, BsmAI, BsmBI, BsmFI, BspMI, BstAPI, DraIII, Earl, FokI, HgaI, PfiMI, SfaNI, and Van91I.
